# EUROPEAN PATENT APPLICATION

(11) **EP 2 228 081 A1**
(43) Date of publication of application: **15.09.2010**
(21) Application number: 07832938.0
(22) Date of filing: 03.12.2007
(51) Int. Cl.: A61L 31/00, A61F 2/82

(54) **STENT AND METHOD OF PRODUCING THE SAME**

(71) Applicant: Goodman Co., Ltd., Nagoya-shi Aichi 465-0032 (JP)
(72) Inventor: KAGAMI, Keiichi, Nagoya-shi Aichi 465-0032 (JP); HINO, Satoki, Nagoya-shi Aichi 465-0032 (JP); NAKAGAWA, Yuki, Nagoya-shi Aichi 465-0032 (JP)
(74) Representative: Winter, Brandl, Fürniss, Hübner, Röss, Kaiser, Polte - Partnerschaft
(86) International application number: PCT/JP2007/073318
(87) International publication number: WO 2009/072172

(57) **Abstract**

Provided is a stent that possesses the rigidity and flexibility required to function as a stent. The stent (10) has a nonwoven fabric structure constituted by fibers (14). More specifically, in this stent, the fibers are oriented either regularly or irregularly, and masses of the fibers are formed from the fibers that are bonded to each other or interlaced either through a mechanical, chemical, thermal, or other procedure or without employing such procedures. The fibers in the stent are constituted by a biodegradable material. Further, the stent is structured such that sheet-form fiber masses having a nonwoven fabric structure are laminated in a radial direction of the stent.

## Description

### TECHNICAL FIELD

The present invention relates to a stent that is inserted into a biological duct for use as an indwelling stent, and a manufacturing method thereof.

### BACKGROUND ART

Conventionally, when a constriction occurs in a biological duct such as an artery, an indwelling cylindrical stent is inserted into the constriction site to secure a channel through the biological duct.

Furthermore, in recent years, various stents using biodegradable material have been proposed. For example, a stent formed by winding a continuous monofilament or multifilament constituted by a biodegradable polymer into a tubular shape while bending the continuous monofilament or multifilament into a zigzag shape has been proposed (see Patent Document 1, for example).

As another example, a stent manufactured by implementing laser treatment or the like on a sheet-form or pipe-form extrudate constituted by a biodegradable polymer has been proposed (see Patent Document 2, for example). Furthermore, a stent formed from a stent base material constituted by knitted, woven, or braided biodegradable fibers has been proposed (see Patent Document 3, for example).
Patent Document 1: WO 00/13737
Patent Document 2: Japanese Translation of PCT Application No. 2007-515249
Patent Document 3: Japanese Patent Application Laid-open No. 2007-130179

However, the stent according to Patent Document 1 is formed by winding linear bodies such as monofilaments, and therefore the stent exhibits superior flexibility but lacks strength. As a result, it may be impossible to realize sufficient radial force (a radial direction force exerted by the stent on an inner wall surface of the biological duct) for the stent to function.

Furthermore, in a case where linear bodies are wound, the linear bodies have a large outer diameter between several tens of µm and several hundred µm, and a small specific surface area. As a result, it is difficult to bring the linear bodies into contact with substances (water, enzymes, and so on, for example) required for decomposition efficiently, and therefore the stent cannot always be said to have sufficient biodegradability.

Meanwhile, the stent according to Patent Document 2 has a base material constituted by an extrudate and is therefore brittle and lacking in elasticity. It may therefore be difficult to obtain sufficient radial force for the stent to function. Further, due to the material quality of the stent, plastic deformation thereof cannot be achieved easily, and it is therefore difficult to make the stent conform to the shape of the biological duct. Accordingly, the stent may contract when attached to a constriction site, thereby returning to its pre-expansion state (i.e. recoiling may occur). Moreover, since the base material is an extrudate, the specific surface area thereof is small, and it may therefore be difficult to advance decomposition.

In the stent according to Patent Document 3, the stent base material is constituted by knitted fibers or the like, and therefore overlapping parts of the fibers may press against a blood vessel inner wall, causing inflammation. Note that in Patent Document 3, the surface of the stent base material is covered by a membranous structure serving as a cell scaffolding material, but an inflammatory response is not always alleviated by this membranous structure. Furthermore, in the case of knitted fibers and so on, the fibers contact each other closely, leading to a reduction in the specific surface area, and it may therefore be difficult for the stent base material to decompose.

### DISCLOSURE OF THE INVENTION

The present invention has been designed in consideration of these circumstances, and a principal object thereof is to provide a novel stent that possesses both the rigidity and the flexibility required to function as a stent, and a manufacturing method thereof. A further object of the present invention is to provide a stent which, when formed using a biodegradable material, exhibits superior biodegradability and adjustability, and a manufacturing method thereof.

To solve the problems described above, the present inventors undertook committed research into stent materials, and as a result of this research, the present inventors discovered that, contrary to expectations, fibrous materials having a nonwoven fabric structure, and in particular fibrous materials formed from nanofibers having a nonwoven fabric structure, are effective as stent materials. More specifically, the present inventors discovered that with a nonwoven fabric structure, design can be performed freely to obtain the radial direction rigidity and axial direction flexibility required for a stent to function, and when a biodegradable material is employed, superior biodegradability derived from the structure can be realized. The present inventors arrived at the present invention on the basis of this knowledge. According to the present invention, the following means are provided.

The present invention provides a stent that is inserted into a biological duct for use as an indwelling stent, wherein the stent has a nonwoven fabric structure constituted by fibers.

This stent is formed from a fiber mass in which the fibers have a nonwoven fabric structure, and is therefore superior in terms of malleability. In other words, mechanical characteristics such as strength and flexibility are set appropriately by bonds or interlacing between the fibers, and therefore the mechanical strength of the stent can be controlled easily by adjusting the orientation, gathering density, and so on of the fibers. Hence, the stent can be patterned freely. As a result, a stent having well-balanced mechanical characteristics can be realized. Accordingly, the stent possesses sufficient radial direction rigidity and axial direction flexibility to function as a stent.

Further, in the present invention, the fibers are preferably constituted by a biodegradable material so as to possess biodegradability.

In this stent, a porous structure is formed by bonding or interlacing the fibers, and therefore a specific surface area of the stent is large. Hence, water, enzymes, and so on are more likely to contact the stent surface, thereby advancing decomposition of the stent within an organism. Therefore, a highly biodegradable stent can be provided. Furthermore, a contact area between the stent and water or the like can be varied easily in accordance with the thickness and gathering density of the fibers, and therefore the biodegradability of the stent can be adjusted easily.

More preferably, at least a part of the fibers is oriented regularly. Even more preferably, the stent is cylindrical and the fibers are oriented in a direction including a circumferential direction component of the stent. At this time, the fibers may also be oriented spirally. When the fibers are oriented in a spiral shape, a tube wall of the stent may have a mesh structure formed by folding back linear bodies to obtain a plurality of folded back portions, and the fibers may be oriented in at least two directions, namely an extension direction of one part and an extension direction of another part on either side of the folded back portions of the linear bodies.

In the stent according to the present invention, the fibers may be formed into a sheet-form fiber mass constituted by the nonwoven fabric structure, the stent may be cylindrical, and the sheet-form fiber mass may form at least a part of the stent by being wound singly or in plurality in the circumferential direction of the stent. In this case, the sheet-form fiber mass preferably forms at least a part of the stent by being laminated in a radial direction of the stent. Further, the sheet-form fiber mass preferably forms at least a part of the stent by means of repeated units constituted by differently oriented fibers.

Furthermore, in the stent according to the present invention, the fibers may be formed into a cylindrical fiber mass constituted by the nonwoven fabric structure, the stent may be cylindrical, and the cylindrical fiber mass may form at least a part of the stent in a state where an axis thereof is aligned with an axis of the stent.

In the present invention, an outer diameter of the fibers is preferably no more than 10 µm. Further, molecular chains constituting the fibers are preferably subjected to at least one of crystallization and orientation. Moreover, the stent may contain a bioactive substance.

The present invention provides a manufacturing method for a stent that is inserted into a biological duct for use as an indwelling stent, including a step of manufacturing a fiber mass having a nonwoven fabric structure constituted by fibers.

In the present invention, the manufacturing method preferably further includes a step of stretching the fibers. Furthermore, the manufacturing step may include the steps of: preparing, as the fiber mass, a sheet-form body having the nonwoven fabric structure constituted by the fibers; and winding one or a plurality of the sheet-form bodies into a cylindrical shape, and forming at least a part of the stent from the fiber mass by setting an axis of the cylindrical fiber mass as an axis of the stent. Alternatively, the manufacturing step may include a step of forming the fiber mass as a cylindrical body having the nonwoven fabric structure constituted by the fibers by discharging the fibers toward a rotary carrier so that the fibers are adhered to the rotary carrier, and forming at least a part of the stent from the fiber mass by setting an axis of the fiber mass as an axis of the stent.

### BRIEF DESCRIPTION OF THE DRAWINGS

Fig. 1 is a schematic diagram showing an example of a stent according to the present invention;
Fig. 2 is an illustrative view showing an example of a stent manufacturing method, wherein 2(a) shows a preparatory step, 2 (b) shows a stretching step, 2 (c) to 2 (e) show a formation step, 2 (f) shows a laminated pipe-form body, and 2 (g) shows the stent; and
Fig. 3 is an illustrative view showing another example of a stent manufacturing method, wherein 3(a) shows a manufacturing step, 3 (b) shows a pipe-form body following fiber gathering, and 3(c) shows the stent.

### EXPLANATION OF REFERENCE NUMERALS

- 10, 20, 30: stent
- 14: fiber
- 16: fiber mass
- 22: sheet-form body serving as sheet-form fiber mass
- 22a: oriented mass serving as sheet-form body piece
- 22b: random mass serving as sheet-form body piece
- 38: cylindrical body serving as cylindrical fiber mass

### BEST MODE FOR CARRYING OUT THE INVENTION

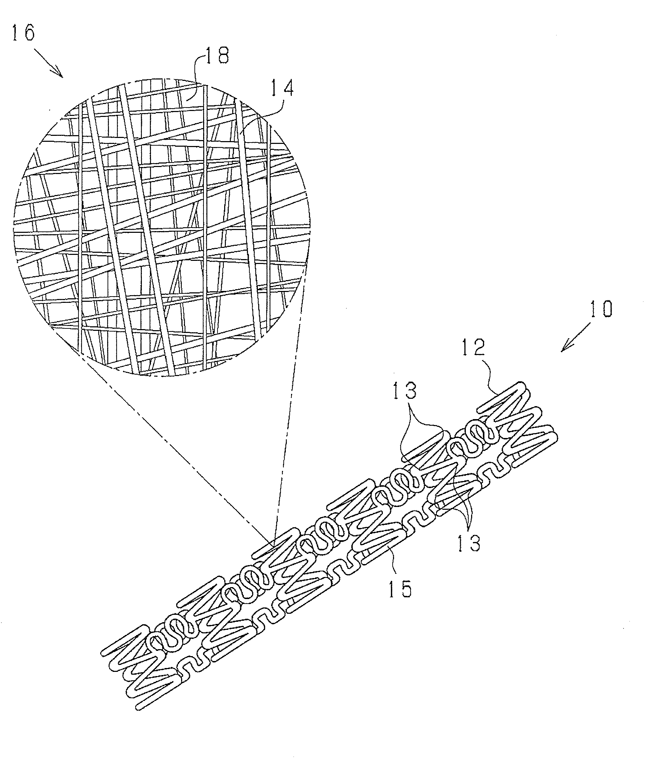

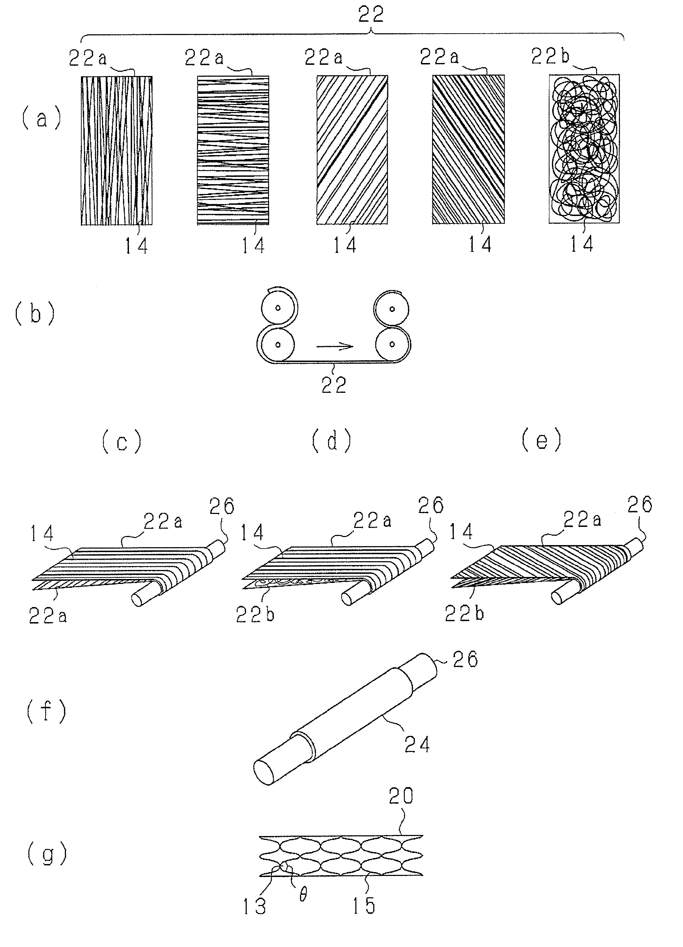

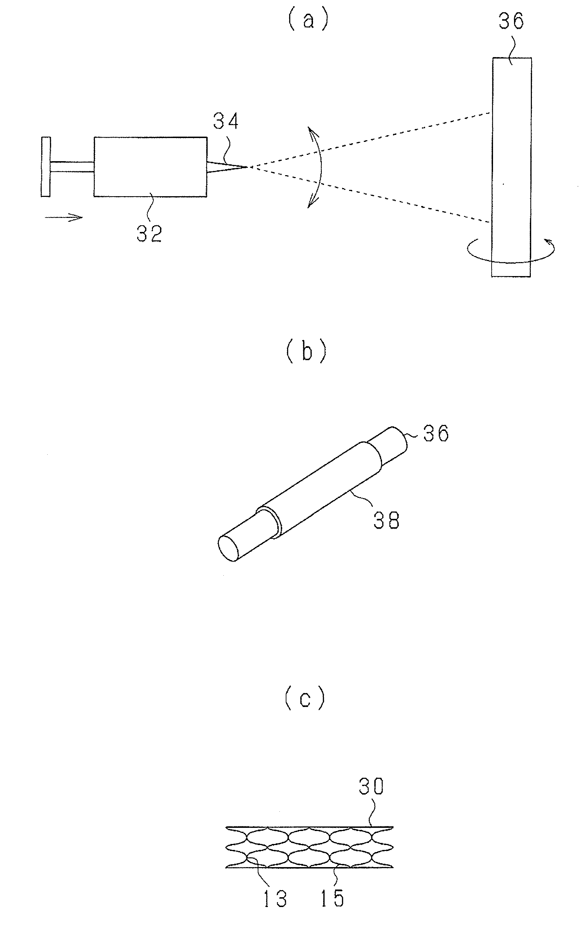

Embodiments of the present invention will be described in detail below with appropriate reference to the drawings. Here, Fig. 1 is a schematic diagram showing an example of a stent according to the present invention, Fig. 2 is an illustrative view showing an example of a stent manufacturing method, and Fig. 3 is an illustrative view showing another example of a stent manufacturing method. Note that the following embodiments are preferred embodiments of the present invention, and the present invention is not limited thereto.

### (Stent)

The stent according to the present invention is inserted into a biological duct for use as an indwelling stent. There are no particular limitations on the biological duct, and the stent may be applied to various biological ducts such as a blood vessel, a trachea, a digestive tract, a urinary duct, an oviduct, or a bile duct, for example. The biological duct is preferably a vessel such as a blood vessel or a lymph duct, and more preferably a blood vessel such as a coronary artery.

The stent may be self-expanding or balloon-expanded but is preferably balloon-expanded. Here, a self-expanding stent is a stent that is inserted into the body while accommodated in a holding body such as a tube in a contracted state. When the holding body is withdrawn from an attachment site, the stent expands under its own restoring force, and as a result, a lumen is secured in the biological duct. A balloon-expanded stent, on the other hand, is a stent that is mounted onto a balloon outer surface in a contracted state and then inserted into the body together with the balloon. The balloon is inflated at the attachment site such that the stent expands, whereupon the stent is separated from the balloon, thereby securing a lumen in the biological duct.

As shown in Fig. 1, a stent 10 has a cylindrical shape and an inside-outside penetrating mesh structure on a tube wall thereof. More specifically, linear bodies 15 are formed on the tube wall of the stent 10, and by disposing a plurality of folded back portions 13 formed by folding back the linear bodies 15 in midway length direction positions on the tube wall, the mesh structure is formed on the tube wall. Note that in Fig. 1, the stent 10 is shown in a condition prior to expansion. Further, in Fig. 1, a region delineated by a circle shows an enlargement of the stent 10.

### (Fibers)

There ae no particular limitations on the material of fibers 14 constituting the stent 10, but a biodegradable material is preferably used. When the fibers 14 are formed from a biodegradable material, the stent 10 dissolves once a fixed time period has elapsed following attachment to the biological duct.

There are no particular limitations on the biodegradable material forming the fibers 14 as long as it is biocompatible. For example, polyester, polyamide, polyurethane, polycarbonate, a copolymer employing two or more of the monomers constituting these materials, and so on may be used. Alternatively, a biological material such as collagen, gelatin, fibrin, albumin, starch, chitosan, or calcium carbonate may be used. Of these materials, polylactic acid (PLLA), +polyglycolic acid (PGA), a polylactic acid- polyglycolic acid copolymer, a polylactic acid-polycaprolactone copolymer, a polylactic acid-polycaprolactone-depsipeptide terpolymer, a poly-D, L-lactide copolymer, a stereocomplex disclosed by the present applicant in Japanese Patent Application Publication No. 2006-175153, and so on are preferable since they contribute to a favorable balance between biodegradability and mechanical characteristics such as strength and flexibility, possess superior biocompatibility, and so on. Further, two or more of the above materials may be used. For example, to increase the toughness of the stent 10, polybutylene succinate or the like may be intermixed with the materials described above.

There are no particular limitations on the outer diameter of the fibers 14, and the outer diameter may be selected appropriately in accordance with the type and diameter of the biological duct to which the stent 10 is to be applied, or the desired biodegradability. For example, when decomposition of the fibers 14 is to be advanced, thin fibers may be used, and when decomposition is to be suppressed, thick fibers may be used. In other words, by setting the outer diameter of the fibers 14 appropriately, the biodegradability of the stent 10 can be controlled easily.

More specifically, the fiber diameter is preferably set substantially in the nano order. The reason for this is that in comparison with a case in which the fiber diameter is larger than the nano order, the specific surface area and the flexibility of the fibers 14 can be increased. As regards a specific numerical value, the fiber diameter may be set at no more than 10 µm, for example. When the fibers 14 are constituted by a biodegradable material with a fiber diameter of no more than 10 µm, the fibers 14 can decompose within a fixed time period (between two and three months, for example). The fiber diameter is preferably set at no less than 3 nm and no more than 5 µm, or more preferably at no less than 100 nm and no more than 1 µm.

### (Nonwoven fabric structure)

In a nonwoven fabric structure according to this specification, masses of the fibers 14 are formed from the fibers that are bonded to each other or interlaced either through a mechanical, chemical, thermal, or other procedure, or without employing such procedures. In other words, in the nonwoven fabric structure, the fibers 14 are regularly or irregularly oriented and then molded into a fiber mass 16 by being overlapped or interlaced without resorting to methods such as knitting, weaving, braiding, or twisting. As a result, the stent 10 possesses superior surface smoothness. Accordingly, the stent 10 does not irritate a wall surface of the biological duct excessively while dwelling in the biological duct, and therefore inflammation in the attachment site of the stent 10 can be suppressed.

There are no particular limitations on the method of forming the nonwoven fabric structure using the fibers 14. For example, a spun bonding method, a flash spinning method, a needle punching method, a melt blowing method, a resin adhesion method, an electro-spinning method, and so on may be employed. Alternatively, the nonwoven fabric structure may be formed by gathering ultrafine fibers manufactured using a laser heating stretching method (disclosed in Japanese Patent No. 3,534,108 or Japanese Patent Application Laid-open No. 2006-57228, for example) on a support. A method with which a nonwoven fabric structure constituted by fibers 14 having a substantially nano order fiber diameter can be formed is preferably employed. More specifically, the electro-spinning method or the laser heating stretching method is preferably employed. The reason for this is that in the fiber mass 16 produced by these methods, the fibers 14 are joined by interlacing, and therefore the flexibility of the stent 10 can be improved.

Of the electro-spinning method and the laser heating stretching method, the electro-spinning method is preferable in terms of productivity, manufacturing cost, and so on. On the other hand, the laser heating stretching method is preferable in terms of the ability to obtain the fibers 14 in a stretched state, the fact that the original yarn does not have to be dissolved in a solvent, and so on.

The nonwoven fabric structure is a porous structure in which a large number of pores 18 are formed between the fibers 14 when the fibers 14 are bonded to each other or interlaced. Therefore, when the fibers 14 are constituted by a biodegradable material, variation in the specific surface area of the stent 10 during decomposition of the stent 10 can be reduced in comparison with a non-porous structure such as that obtained when the base material is an extrudate or the like. As a result, a decomposition rate of the stent 10 can be kept constant.

Furthermore, the specific surface area of the stent 10 is increased by the porous structure, and therefore water, enzymes, and so on enter the pores 18 easily. Hence, when the fibers 14 are constituted by a biodegradable material, decomposition of the stent 10 can be advanced easily. When the fibers 14 have a substantially nano order outer diameter, the specific surface area of the fibers 14 is extremely large, and therefore decomposition of the stent 10 is advanced particularly easily. Moreover, when the stent 10 is applied to a blood vessel, for example, vascular endothelial cells infiltrate the lumen of the stent 10 easily through the pores 18. The vascular endothelial cells then adhere to the inner wall surface of the stent 10 such that the blood vessel is reconstructed more easily. As a result, a thrombosis can be prevented from forming in the constriction site.

There are no particular limitations on the size (fiber density) of the pores 18, and various sizes may be set in accordance with the desired degree of biodegradability and so on. In other words, the degree of biodegradability to be applied to the stent 10 can be adjusted by varying the size of the pores 18. More specifically, the fibers 14 are gathered loosely to enhance the biodegradability and gathered densely to suppress the biodegradability.

### (Fiber orientation)

The fibers 14 of the nonwoven fabric structure may be oriented either regularly or irregularly, and this determination may be made appropriately in accordance with the mechanical characteristics to be applied to the stent 10. Here, "irregularly oriented" means that the fibers 14 are gathered randomly, while "regularly oriented" includes an embodiment in which the fibers 14 are gathered in a single direction and a multi-layer embodiment in which the fibers 14 are gathered in one or a plurality of directions on top of fibers 14 gathered in a different single direction. For example, when the fibers 14 are oriented irregularly, the overall rigidity of the stent 10 can be improved. When the fibers 14 are oriented regularly such that the fibers 14 have a directional property, on the other hand, the rigidity of the stent 10 with respect to a desired direction can be improved.

The fibers 14 preferably include a regular orientation. The regular orientation may be unidirectional but is preferably multidirectional. Thus, cracks can be prevented from occurring in the orientation directions of the fibers 14, and the toughness of the stent 10 can be improved. Moreover, an improvement in strength can be achieved in relation to multiple directions, and the strength of the stent 10 can be controlled freely.

In a preferred embodiment, the fibers 14 are oriented more densely in a direction including at least one of an axial direction component and a circumferential direction component of the stent 10. By orienting the fibers 14 in the axial direction of the stent 10, the rigidity of the stent 10 against axial direction bending can be improved. By orienting the fibers 14 in the circumferential direction, the rigidity of the stent 10 against radial direction force can be improved. In particular, the stent 10 requires both axial direction flexibility and radial direction rigidity in order to function as a stent, and therefore the fibers 14 are preferably oriented more densely in the circumferential direction than in the axial direction.

In this case, the fibers 14 are preferably gathered in a combination of a regular orientation and an irregular orientation. By adding the irregularly oriented fibers 14, the overall strength of the stent 10 can be improved while maintaining the mechanical characteristics derived from the directional property of the fibers 14. A more preferable embodiment includes fibers 14 oriented in the circumferential direction and irregularly oriented fibers 14. In this case, sufficient strength and radial force for the stent 10 to function can be obtained, and in addition, an appropriate degree of flexibility can be exhibited.

In another preferred embodiment, the fibers 14 are oriented spirally about the axis of the stent 10. More preferably, a spiral shape formed by the fibers 14 is appended to the tube wall of the stent 10 in two or more different directions. There are no particular limitations on the directions of the fibers 14 in this case, but the fibers 14 are preferably oriented in symmetry with the axis of the stent 10. In so doing, a favorable balance of rigidity in relation to the axial direction component and the circumferential direction component of the stent 10 can be achieved.

In a case where the fibers 14 are oriented spirally, there are no particular limitations on an intersection angle between the fibers 14 oriented in the different directions. The intersection angle is preferably substantially identical to an angle (an opening angle 8) formed by one extension direction component and another extension direction component on either side of the folded back portions 13 of the mesh structure (for example, a zigzag shape, a sine wave shape, or similar) formed on the tube wall of the stent 10. Thus, when the stent 10 contracts or expands, the fibers 14 are oriented in an expansion/contraction direction of the linear bodies 15 of the stent 10, and therefore the stent 10 deforms easily at the folded back portions 13 without losing its pre-contraction structure. Hence, during contraction and expansion of the stent 10, the stent 10 returns to its original, pre-contraction shape easily. In other words, when the stent 10 is placed in a biological duct, the stent 10 can dwell in its original, pre-contraction shape. As a result, the mechanical characteristics of the stent 10 can be exhibited sufficiently, and the lumen of the biological duct can be secured appropriately. There are no particular limitations on the specific opening angle θ, but in a case where fibers 14 in two different directions are formed into a spiral shape, the opening angle θ may be set at no less than 40° and no more than 80°. Preferably, the opening angle θ is set at no less than 50° and no more than 70°.

### (Embodiments of fiber mass)

There are no particular limitations on the manner in which the stent 10 is formed by the fibers 14. In one embodiment, a fiber mass 16 (fiber web) having a web structure constituted by the fibers 14 may be employed. More specifically, the fiber web is formed in a cylindrical shape, whereupon the cylindrical fiber web is compacted by implementing heat treatment or the like, for example, to form a cylindrical body serving as the stent 10. In this embodiment, a stent 10 in which the fibers 14 are oriented irregularly can be obtained.

In another embodiment, the fiber mass 16 may be formed into a sheet-form body constituted by the fibers 14, whereupon the sheet-form body is rolled into a cylindrical shape to obtain a cylindrical body serving as the stent 10. In this case, the cylindrical body may be formed from a single thick sheet-form body having a certain degree of thickness, but a cylindrical body having a multi-layer structure is preferably formed by winding one or a plurality of thin sheet-form bodies into a scroll shape. In this case, when the fibers 14 constituting the sheet-form bodies are oriented regularly, the regular orientation of the fibers 14 can be incorporated into the stent 10. An increase in the thickness of the stent 10 at joint portions where end portions of the sheet-form bodies are joined together can be suppressed by displacing the joint portions.

When a multi-layer structure is employed, the structure preferably includes repeated units constituted by differently oriented fibers 14. For example, when the fibers 14 are oriented in two different directions, two types of sheet-form bodies having different orientations are laminated alternately. In so doing, sufficient strength for the stent 10 to function can be obtained and post-expansion recoiling can be suppressed. For example, to include repeated units in the orientation of the fibers 14, a plurality of small pieces serving as sheet-form bodies may be prepared, whereupon the small pieces are laminated in sequence, one or a plurality of pieces at a time, while ensuring that the fibers 14 are oriented regularly. Preferably, however, a plurality of rectangular sheet-form bodies are overlapped such that the fibers 14 are oriented differently, whereupon the sheet-form bodies are wound into a scroll shape. As a result, the number of joint parts between the sheet-form bodies can be reduced and repeated units can be formed easily in the orientation.

Further, when the nonwoven fabric structure is formed by interlacing the fibers 14, flexibility derived from the structure is combined with the orientation (directional property) of the fibers 14, and therefore the rigidity and flexibility required by the stent 10 can be realized in the stent 10. More specifically, in a case where the fibers 14 are interlaced, a favorable balance can be obtained in the stent 10 between axial direction flexibility and circumferential direction rigidity by orienting the fibers 14 more densely in the circumferential direction.

When the electro-spinning method is used to obtain the sheet-form bodies constituted by the fibers 14, for example, a discharge port (a nozzle) for discharging a fiber solution of the dissolved fibers 14 is placed opposite a substrate and a discharge liquid is adhered to the substrate from the discharge port. As a result, the fiber mass 16 is formed on the substrate. The sheet-form body formed from the fibers 14 can then be obtained by peeling the fiber mass 16 away from the substrate surface. When, at this time, the fiber solution is adhered to a substrate constituted by a rotary body, the fibers 14 can be oriented regularly on the sheet-form body. More specifically, a spinneret is provided in a position facing an outer peripheral surface or an inner peripheral surface of the rotary body substrate, whereupon the fiber solution is adhered to the outer peripheral surface or the inner peripheral surface of the substrate while rotating the substrate axially. As a result, a fiber mass 16 formed from fibers 14 oriented in an identical direction to a rotation direction of the substrate is wound onto the substrate. A sheet-form body on which the fibers 14 are oriented regularly can then be obtained by peeling the fiber mass 16 away from the substrate.

Alternatively, when the laser heating stretching method is employed, the sheet-form body constituted by the fibers 14 can be formed by gathering manufacturing or semi-manufactured fibers 14 on a substrate. By gathering the fibers 14 such that the fibers 14 possess a directional property at this time, a sheet-form body on which the fibers 14 are oriented regularly can be obtained.

In a further embodiment of the fiber mass 16, a cylindrical fiber mass 16 may be formed from the fibers 14, and a cylindrical body serving as the stent 10 may be formed by aligning an axis of the cylindrical body with the axis of the stent 10. More specifically, fibers 14 that have been manufactured or semi-manufactured using the electro-spinning method, the laser heating stretching method, or another method are adhered to an outer peripheral surface or an inner peripheral surface of a rotary carrier, whereupon the rotary carrier is removed to obtain a cylindrical body serving as the stent 10 constituted by the fibers 14. This embodiment is particularly favorable for orienting the fibers 14 in the circumferential direction of the stent 10. Moreover, in contrast to the sheet-form body, joint portions need not be provided on the ends, and therefore the fibers 14 can be gathered substantially evenly in the circumferential direction of the stent 10. As a result, a surface smoothness of the stent 10 can be improved. Furthermore, in this embodiment, the fibers 14 can be oriented in an arbitrary single direction (the axial direction, for example) of the stent 10 by adhering the fibers 14 to the rotary carrier while causing the fibers 14 to reciprocate.

Note that web-form, sheet-form and cylindrical fiber masses 16 may be used singly or combined in plurality to form the stent 10 using the fibers 14. Further, if necessary, a pipe-form body constituted by the fiber masses 16 may be compacted subsequently by compressing the pipe body while applying heat.

### (Stretching)

The fibers 14 are preferably molded into the stent 10 in a stretched state. When stretching is employed, molecular chains constituting the fibers 14 are highly oriented (molecular orientation), and therefore the strength of the fibers 14 can be improved. When the fibers 14 are generated using the laser heating stretching method, the fibers 14 are collected in a stretched state, and therefore labor expended on stretching can be eliminated. When the fibers 14 are prepared using the electro-spinning method, on the other hand, the fibers 14 are in a non-stretched state (an amorphous state), and therefore stretching is required. There are no particular limitations on the stretching method employed in this case. To obtain fibers 14 that are highly oriented and have a high crystallization speed in a case where the fibers 14 are constituted by a crystalline polymer, the temperature during stretching is preferably set between a glass transition temperature and a crystallization temperature of the material constituting the fibers 14. Note that the molecular chain orientation may be analyzed using X-ray analysis, Fourier transform infrared (FTIR) spectroscopy, or another method, for example.

As long as the stent 10 is constituted by the stretched fibers 14, stretching may be performed at any stage. More specifically, stretching may be performed before the fiber mass 16 is formed from the fibers 14 or after the fiber mass 16 has been formed in a sheet shape, a cylindrical shape, or another shape using non-stretched fibers 14.

Further, in the case of a crystalline polymer, the molecular chains of the fibers 14 are preferably crystallized. In so doing, the strength of the fibers 14 can be increased and the fibers 14 can be provided with a superior mechanical property. To crystallize the molecular chains, the fibers 14 may be stretched at a temperature between the glass transition temperature and the crystallization temperature, for example. Alternatively, the molecular chains may be oriented in the vicinity of the glass transition temperature and then subjected to heat treatment in the vicinity of the crystallization temperature. Note that the crystallinity of the molecules may be analyzed using differential scanning calorimetry (DSC), a transmission electron microscope (TEM), an atomic force microscope (AFM), an X-ray diffractometer, and so on.

After forming the fiber mass 16 into a cylindrical body, an inside-outside penetrating mesh structure is cut into a tube wall of the cylindrical body by laser processing using an excimer laser or the like, etching, a cutting tool, or another method, for example. The fiber mass 16 can be handled as a molded component and is therefore subject to few design restrictions. In other words, when the mesh is cut into the cylindrical body serving as the fiber mass 16, various mesh forms with which the mechanical characteristics required for the stent 10 to function, i.e. axial direction flexibility and radial direction rigidity, may be employed. As a result, a high-performance stent 10 exhibiting superior strength and flexibility can be manufactured. Further, the fibers 14 are formed from a nonwoven fabric structure in which the fibers 14 are integrated by interlacing or the like, and therefore, in contrast to a knitted fabric or the like, the fibers 14 can be prevented from unraveling and separating even when a mesh structure having a complicated shape is applied. Hence, even when the mesh structure has a complicated shape, the shape can be maintained sufficiently. When the outer diameter of the fibers 14 is substantially in the nano order, the fiber diameter is extremely small, and therefore the fibers 14 can be interlaced particularly easily. As a result, the shape of the fiber mass 16 can be maintained easily.

The stent 10 constituted in this manner may be caused to contract after being subjected to shape memory treatment or the like to the size thereof when dwelling in the biological duct, for example. In this case, the stent 10 can function as a balloon-expanded stent.

### (Bioactive substance)

As long as the basic stent 10 has a nonwoven fabric structure constituted by the fibers 14, the stent 10 may contain a compound other than the fibers 14, for example an additive, a bioactive substance, or the like, provided the characteristics of the stent 10 are not tainted thereby.

Various pharmaceuticals and the like may be applied as the bioactive substance. A pharmaceutical that suppresses restenosis in the dwelling site of the stent 10 is preferably employed. Specific examples of this type of pharmaceutical include an anti-cancer agent, an immunosuppressant, an antibiotic, an antithrombotic agent, an HMG-CoA reductase inhibitor, an ACE inhibitor, a calcium antagonist, a hypolipidemic agent, a lipid enhancer, a DNA synthesis inhibitor, a vascular smooth muscle proliferation inhibitor, an anti-inflammatory agent, and an interferon.

There are no particular limitations on the manner in which the bioactive substance is included in the stent 10. For example, the stent 10 may be coated with the bioactive substance, or the bioactive substance may be chemically bonded to the molecules constituting the fibers 14 or the like. When coating is employed, the stent 10 may be coated with a simple bioactive substance or with a biodegradable polymer having a different decomposition rate to the stent 10. Alternatively, the bioactive substance may be carried in the pores 18.

### (Stent manufacturing method)

A stent manufacturing method according to the present invention may be used to manufacture the stent 10 according to the present invention described above, for example. According to this manufacturing method, a stent can be manufactured using the fiber mass 16, which exhibits superior malleability, and therefore a high-performance stent possessing both radial direction rigidity and axial direction flexibility can be obtained. An example of this stent manufacturing method is shown in Fig. 2.

### (Preparatory step)

As shown in Fig. 2(a), in a preparatory step of the manufacturing method, first, a sheet-form fiber mass (sheet-form body) 22 formed by gathering the fibers 14 described above is manufactured. The sheet-form body 22 may be manufactured by forming an oriented mass 22a in which the fibers 14 are oriented in a single direction and a random mass 22b in which the fibers 14 are oriented randomly either singly or in plurality depending on the design of a stent 20 and so on.

### (Stretching step)

Next, as shown in Fig. 2(b), the sheet-form body 22 is preferably stretched at a temperature between the glass transition temperature and the crystallization temperature of the fibers 14, for example. At this time, primary stretching alone or both primary stretching and secondary stretching may be performed. Further, there are no particular limitations on the stretching method, and roll stretching or tenter stretching, for example, may be employed. Note that the stretching step may be performed after the sheet-form body 22 is formed into a cylindrical body in a subsequent formation step by expanding the cylindrical body in the radial direction, for example. The cylindrical body may be expanded by disposing a balloon in a lumen formed in the cylindrical body and then expanding the balloon, for example. Alternatively, a resin tube may be inserted into the lumen and expanded in the radial direction by blow molding the tube. Alternatively, the sheet-form body 22 may be formed from the fibers 14 after performing the stretching step on the fibers 14 during or after spinning of the fibers 14.

### (Formation step)

As shown in Figs. 2 (c) to 2 (e), after the preparatory step or the stretching step following the preparatory step, the sheet-form body 22 obtained in the preparatory step is formed into a cylindrical shape by being wound around a cylindrical carrier 26 in a circumferential direction, for example. As regards a method for forming the sheet-form body 22 into a cylindrical shape, the sheet-form bodies 22 may be wound around the carrier 26 one at a time, but preferably, a plurality of the sheet-form bodies 22 are overlapped and then formed into a cylindrical shape. At this time, the sheet-form bodies 22 are preferably laminated taking the orientation of the fibers 14 constituting the sheet-form bodies 22 into consideration. For example, the fibers 14 may be oriented in the axial direction and circumferential direction of the carrier 26, as shown in Fig. 2(c), or regularly and irregularly oriented fibers 14 may be combined, as shown in Fig. 2(d). Alternatively, as shown in Fig. 2(e), the fibers 14 may be oriented such that the fibers 14 depict a spiral shape relative to the axis of the carrier 26. The end portions of the sheet-form bodies 22 may be joined one sheet-form body 22 at a time using an adhesive or the like, for example, but are preferably joined by interlacing the fibers 14. The joint portions of the respective sheet-form bodies 22 are preferably laminated so as not to overlap.

An obtained cylindrical body 24 (see Fig. 2(f)) may be compacted by applying heat treatment thereto. The cylindrical body 24 is then separated from the carrier 26, whereupon a mesh structure is formed on the entire cylindrical body 24 by laser processing or the like, for example. As a result, the stent 20 shown in Fig. 2(g) is completed.

In another example of the manufacturing method, a cylindrical fiber mass 16 (cylindrical body) formed from a nonwoven fabric structure constituted by the fibers 14 may be manufactured as the fiber mass 16 instead of the sheet-form body 22 such that the stent is manufactured using this cylindrical body. More specifically, as shown in Fig. 3, a fiber solution charged into a syringe 32 is discharged from a nozzle 34 toward a rotary carrier 36 so as to be adhered to the rotary carrier 36, and thus a cylindrical body 38 serving as the fiber mass 16 is manufactured.

In more detail, first, as shown in Fig. 3(a), the rotary carrier 36 and the nozzle 34 are positioned such that the nozzle 34 faces an outer surface of the rotary carrier 36. The fiber solution ejected from the nozzle 34 is then adhered to the outer surface of the rotary carrier 36 as the rotary carrier 36 is rotated in the circumferential direction thereof. At this time, when a rotation speed of the rotary carrier 36 is low, the fibers 14 are oriented randomly on the outer surface of the rotary carrier 36, and when the rotation speed is high, the fibers 14 are oriented on the outer surface of the rotary carrier 36 in the circumferential direction thereof. Further, by ejecting the fiber solution while causing the syringe 32 to reciprocate in the axial direction of the rotary carrier 36, the fibers 14 can be oriented in the axial direction of the rotary carrier 36.

The cylindrical body 38 (see Fig. 3 (b)) obtained in this manner is removed from the rotary carrier 36, whereupon a stent 30 (see Fig. 3(c)) is formed by creating a mesh structure on the entire cylindrical body 38 using laser processing or the like, for example.

Note that the stents 20, 30 may be formed from a combination of the sheet-form body 22 and the cylindrical body 38 serving as the fiber masses 16. More specifically, a body obtained by forming the sheet-form bodies 22 into a cylindrical shape may be used as the rotary carrier 36 shown in Fig. 3, and additional fibers 14 may be gathered on the surface of the rotary carrier 36 by ejecting the fiber solution onto a circumferential surface of the rotary carrier 36. Alternatively, one or a plurality of additional sheet-form bodies 22 may be wound around an outer peripheral surface of the cylindrical body 38. Examples

Specific examples of the present invention will be described below. However, the present invention is not limited to the specific examples to be cited below.

### (1) Manufacture of fiber sheet

To manufacture a fiber sheet, a polymer solution was subjected to electro-spinning using an electro-spinning device (Nanon-01A, manufactured by MEC Co., Ltd.). The polymer solution was prepared by dissolving polylactic acid (PLLA) having a weighted average molecular weight of approximately 80,000 in hexafluoroisopropanol (HFIP) such that the PLLA was 15 wt%. An applied voltage was applied while being adjusted appropriately between 10 and 30 kev.

The generated fibers were then wound around an outer peripheral surface of a rotating drum having a diameter of 10 cm. A drum rotation speed was set between 2000 and 2500 rpm to manufacture an oriented sheet and at no more than approximately several hundred rpm to manufacture an irregular sheet. The sheet was formed at an even thickness by causing the drum to reciprocate in an axial direction of the drum at this time.

The obtained fiber sheet had a film thickness between 60 and 70 µm. Further, the oriented sheet was formed with an opaque white gloss. The oriented sheet was strong at bends formed in a curve direction of the fibers, and flexible but more likely to split at bends in an extension direction of the fibers. Meanwhile, the irregular sheet was an opaque white sheet having a smooth surface, and possessed flexibility at bends in all directions. After observing the obtained fiber sheets using an electron microscope, it was found that both the oriented sheet and the irregular sheet had a fiber diameter of 600 to 700 nm. It was also confirmed that in the oriented sheet, the fibers were cleanly oriented in a substantially single direction.

### (2) Manufacture of stent

The obtained fiber sheets were cut to a predetermined size (width 20 mm, length 50 to 250 mm) and then wound around a mandrel (diameter 1.0 to 1.2 mm). At this time, five types of tube bodies (fiber tubes) having different fiber orientations were manufactured. More specifically, a body formed by winding a single irregular sheet (length 100 mm) (first experimental example), a body formed by winding a single oriented sheet (length 100 mm) in which the fibers were oriented in the circumferential direction (second experimental example), a body formed by winding one irregular sheet (length 50 cm) and one oriented sheet (length 50 cm, fibers oriented in the axial direction) in an overlapped state (third experimental example), a body formed by winding one oriented sheet (length 50 cm, fibers oriented in the circumferential direction) and one oriented sheet (length 50 cm, fibers oriented in the circumferential direction) in an overlapped state (fourth experimental example), and a body formed by overlapping two sheets (length 250 mm) obtained by cutting the oriented sheet at an angle of 30° to a winding axis such that an angle of intersection between the fibers was 60° (120°) and then winding the overlapped sheets (fifth experimental example) were manufactured. Note that in the fifth experimental example, the manufactured fiber sheets were stretched in advance (to a film thickness of 10 to 15 µm) and then wound around a mandrel (diameter 3 mm).

A thermal shrink tube (which thermally shrinks at or above 90 to 120°C) was placed on the wound fiber sheets to cover the sheets entirely, whereupon heat was applied for 10 minutes at 110 to 120°C. As a result, the thermal shrink tube was caused to shrink, thereby forming the fiber sheets into a tubular shape.

A polyamide tubular body was inserted into the lumen of the fiber tube, whereupon fiber stretching was performed by blow-molding the tubular body at 90 degrees such that the inner diameter and outer diameter of the fiber tube were 3.0 mm and 3.4 mm, respectively. Note that in the fifth experimental example, stretching was performed in advance, and therefore stretching was not performed here. The tube wall of the fiber tube was then cut into a mesh structure using an excimer laser, and thus five types of stents having different fiber orientations were created. The mesh structure was formed in a zigzag lattice shape in which the folded back portions of the linear bodies forming the mesh were rounded. In the fifth experimental example, an opening angle θ of the folded back portions of the linear bodies (see Fig. 2 (g)) was substantially aligned with the angle of intersection between the fibers. All of the obtained stents had an inner diameter of 3.0 mm and an outer diameter of 3.4 mm. The fiber orientations of the respective stents are shown in Table 1.

**[Table 1]**

| | Fiber orientation | Functional evaluation |
|---|---|---|
| First experimental example | Irregular | △ |
| Second experimental example | Circumferential direction | △ |
| Third experimental example | Irregular + circumferential direction | ⊚ |
| Fourth experimetal example | Circumferential direction + axial direction | ○ |
| Fifth experimental example | Spiral | ⊚ |

| | | |
|---|---|---|
| ⊚ Favorably ○ Medium △ Rather poor | | |

### (3) Functional evaluation of stents

A functional evaluation was performed on the various stents shown in Table 1. In the functional evaluation, the radial force and the flexibility of the stents were compared. More specifically, the respective stents were caused to contract and then mounted on a PTCA balloon catheter, whereupon the balloon catheter was expanded. The resulting expanded stents were then pressed using a finger and functionally evaluated through touch.

It was possible to expand all of the stents shown in Table 1 without problems, and all of the stents had an appropriate degree of flexibility. Further, little post-expansion contraction (recoil) occurred. As regards the radial force, the third and fifth experimental examples were most favorable. The fourth experimental example possessed sufficient radial force, albeit less than the third and fifth experimental examples. The first and second experimental examples were slightly deficient in radial force.

It was learned from these results that a stent formed from fibers having a nonwoven fabric structure possesses the rigidity and flexibility required to function as a stent. It was also learned that when the fibers are oriented in a plurality of different directions, a particularly favorable stent exhibiting superior characteristics such as strength, radial force, and flexibility and little post-expansion recoil is obtained. It was also learned that, among the manufactured stents, a stent including both a circumferential direction orientation and an irregular orientation and a stent in which the fibers are oriented in a spiral shape extending in a plurality of different directions are particularly favorable.

## Claims

1. A stent that is inserted into a biological duct for use as an indwelling stent, **characterized in that** the stent has a nonwoven fabric structure constituted by fibers.

2. The stent according to claim 1, **characterized in that** the fibers are constituted by a biodegradable material and therefore possess biodegradability.

3. The stent according to claim 1 or 2, **characterized in that** at least a part of the fibers is oriented regularly.

4. The stent according to any of claims 1 to 3, **characterized in that** the stent is cylindrical, and
the fibers are oriented in a direction including a circumferential direction component of the stent.

5. The stent according to claim 4, **characterized in that** the fibers are oriented spirally.

6. The stent according to claim 5, **characterized in that** a tube wall of the stent has a mesh structure formed by folding back linear bodies to obtain a plurality of folded back portions, and
the fibers are oriented in at least two directions, namely an extension direction of one part and an extension direction of another part on either side of the folded back portions of the linear bodies.

7. The stent according to any of claims 1 to 6, **characterized in that** the fibers are formed into a sheet-form fiber mass constituted by the nonwoven fabric structure,
the stent is cylindrical, and
the sheet-form fiber mass forms at least a part of the cylindrical stent by being wound singly or in plurality in the circumferential direction of the stent.

8. The stent according to claim 7, **characterized in that** the sheet-form fiber mass forms at least a part of the stent by being laminated in a radial direction of the stent.

9. The stent according to claim 7 or 8, **characterized in that** the sheet-form fiber mass forms at least a part of the stent by means of repeated units constituted by differently oriented fibers.

10. The stent according to any of claims 1 to 9, **characterized in that** the fibers are formed into a cylindrical fiber mass constituted by the nonwoven fabric structure,
the stent is cylindrical, and
the cylindrical fiber mass forms at least a part of the stent in a state where an axis thereof is aligned with an axis of the stent.

11. The stent according to any of claims 1 to 10; **characterized in that** an outer diameter of the fibers is no more than 10 µm.

12. The stent according to any of claims 1 to 11, **characterized in that** molecular chains constituting the fibers are subjected to at least one of crystallization and orientation.

13. The stent according to any of claims 1 to 12, **characterized in that** the stent contains a bioactive substance.

14. A manufacturing method for a stent that is inserted into a biological duct for use as an indwelling stent, **characterized by** comprising a manufacturing step of manufacturing a fiber mass having a nonwoven fabric structure constituted by fibers.

15. The manufacturing method for a stent according to claim 14, **characterized by** further comprising a stretching step of stretching the fibers.

16. The manufacturing method for a stent according to claim 14 or 15, **characterized in that** the manufacturing step comprises the steps of:
preparing a sheet-form body having the nonwoven fabric structure constituted by the fibers, as the fiber mass; and
winding at least one of the sheet-form bodies into a cylindrical shape, and forming at least a part of the stent from the fiber mass by setting an axis of the cylindrical fiber mass as an axis of the stent.

17. The manufacturing method for a stent according to any of claims 14 to 16, **characterized in that** the manufacturing step comprises a step of forming the fiber mass as a cylindrical body having the nonwoven fabric structure constituted by the fibers by discharging the fibers toward a rotary carrier so that the fibers are adhered to the rotary carrier, and forming at least a part of the stent from the fiber mass by setting an axis of the fiber mass as an axis of the stent.
